# EUROPEAN PATENT APPLICATION

(11) **EP 3 498 293 A1**
(43) Date of publication of application: **19.06.2019**
(21) Application number: 17306783.6
(22) Date of filing: 15.12.2017
(51) Int. Cl.: A61K 39/395, C07K 16/28

(54) **TREATMENT OF MONOGENIC DISEASES WITH AN ANTI-CD45RC ANTIBODY**

(71) Applicant: INSTITUT NATIONAL DE LA SANTE ET DE LA RECHERCHE MEDICALE (INSERM), 75013 Paris (FR); Universite de Nantes, 44000 Nantes (FR); Chu Nantes, 44000 Nantes (FR)
(72) Inventor: GUILLONNEAU, Carole, 44118 LA CHEVROLIERE (FR); ANEGON, Ignacio, 44000 NANTES (FR)
(74) Representative: Icosa

(57) **Abstract**

The present invention relates to an anti-CD45RC antibody for use in the treatment of monogenic diseases wherein genes involved in said monogenic diseases are:
- genes which are not associated with immune function but whose deficiency is associated with inflammation and/or immune reactions, such as genes deficient in the following diseases: Duchenne muscular dystrophy (DMD), cystic fibrosis, lysosomal diseases and alpha1-anti-trypsin deficiency; or
- genes involved in the immune system and whose deficiency generates inflammation and/or autoimmune reactions, such as genes deficient in the following diseases: T-cell primary immunodeficiencies such as IPEX (immunodysregulation polyendocrinopathy enteropathy X-linked syndrome), APECED (autoimmune polyendocrinopathy-candidiasis-ectodermal dystrophy), B cell primary immunodeficiencies, Muckle-Wells syndrome, Mixed autoinflammatory and autoimmune syndrome, NLRP12-associated hereditary periodic fever syndrome, and Tumor necrosis factor receptor 1 associated periodic syndrome.

The present invention is also directed to a composition and a kit comprising said anti-CD45RC antibody and an immunosuppressive and/or anti-inflammatory drug.

SEQUENCE LISTING

<110>  INSERM
       UNIVERSITE DE NANTES
       CHU NANTES

<120>  TREATMENT OF MONOGENIC DISEASES WITH AN ANTI-CD45RC ANTIBODY

<130>  D37099 B73511

<160>  2

<170>  PatentIn version 3.5

<210>  1
<211>  144
<212>  DNA
<213>  Homo sapiens

<400>  1
atgtcccagg agagaggagt acagccagca cctttcctac agacccagtt tccccattga      60

caaccaccct cagccttgca caccacagct ctgctgcctt acctgcacgc acctccaaca     120

ccaccatcac agcgaacacc tcag                                            144


<210>  2
<211>  47
<212>  PRT
<213>  Homo sapiens

<400>  2

Cys Pro Arg Arg Glu Glu Tyr Ser Gln His Leu Ser Tyr Arg Pro Ser
1               5                   10                  15


Phe Pro Ile Asp Asn His Pro Gln Pro Cys Thr Pro Gln Leu Cys Cys
            20                  25                  30


Leu Thr Cys Thr His Leu Gln His His His His Ser Glu His Leu
        35                  40                  45

## Description

The present invention is in the field of immunotherapy in the field of genetic diseases. More particularly, the invention relates to an isolated anti-CD45RC antibody for use in preventing or treating monogenic diseases such as Duchenne muscular dystrophy (DMD).

Monogenic diseases are caused by single-gene defects. Over 4000 human diseases are caused by these defects linked to one particular gene. Up to now, most treatment options revolve around treating the symptoms of the disorders, in an attempt to improve patient quality of life. Gene therapy, i.e. a form of treatment where a healthy gene is introduced into the body of a patient, is the main hope for durable treatments of this type of diseases. However, major obstacles have been encountered during the development of techniques for the delivery of genes to the appropriate cells affected by the disorder.

Among monogenic diseases, some are linked to genes involved in the immune system, or to genes not associated with immune functions but whose deficiency is associated with inflammation and/or immune reactions. Among them, the present application focuses on Duchenne muscular dystrophy (DMD), and on the autoimmune polyendocrinopathy-candidiasis-ectodermal dystrophy (APECED).

DMD is a monogenic disease wherein mutations of the *DMD* gene coding for the protein dystrophin lead to severe X-linked muscular dystrophy, which affects all voluntary muscles as well as the heart and breathing muscles in later stages.

Immune responses are involved in the pathophysiology of disease in both DMD patients and *mdx* mice (for a review see Rosenberg et al., 2015). Standard treatment of DMD patient are corticoids, such as prednisolone, and in *mdx* mice treatments decreasing effector immune responses or inflammation, such as intravenous immunoglobulins, tranilast, heme oxygenase-1 inducers, IL-1 receptor antagonist and IL-2 to amplify regulatory T cells (T_{regs}) (Rosenberg et al., 2015; Villalta et al. 2014).

However, despite recent promising new treatments, the average life expectancy of DMD patients is still severely reduced. Hence what is needed is a new treatment to reduce or inhibit immune responses in DMD and in related diseases.

Autoimmune diseases are caused by breakdown of self-tolerance that leads to a dysfunction of the immune system and is the cause of serious, disabling and even fatal consequences. A better knowledge of immunological mechanisms involved in tolerance represents a major challenge to improve both the understanding and treatment of autoimmune diseases.

A key player in this equilibrium is AIRE, a transcription regulator that allows the expression of tissue restricted antigens (TRA) in medullary epithelial thymic cells (mTECs) and auto-reactive T cells deletion.

The autoimmune polyendocrinopathy-candidiasis-ectodermal dystrophy (APECED) also known as auto-immune polyglandular syndrome type I (APS 1) is a rare multi-organ autosomal recessive auto-immune disease caused by mutations in the *AIRE* gene.

In human, this gene is located on locus 21q22.3 and more than 100 mutations have been described to cause APECED with a prevalence of 1-9: 1,000,000 (Orphanet, http://www.orpha.net), this prevalence being increased in certain populations such as Finnish, Norwegian, Sardinian and Iranian Jews. Among Sardinians, the over representation of the R139X mutation is observed, present for instance in 90% of Sardinian APECED patients.

The clinical phenotype of APECED is usually defined by the presence of 2 of the 3 major symptoms: hypoparathyroidism, adrenal insufficiency (Addison's disease) and chronical muco-cutaneous candidiasis (CMC). This disease is also associated with multiple autoimmune and ectodermal features such as type 1 diabetes, enamel hypoplasia, vitiligo, premature ovarian failure, keratitis, pernicious anemia, alopecia, exocrine pancreatitis, interstitial lung disease, nephritis and other disorders.

### DESCRIPTION

The inventors demonstrated that treatment of *Dmd^{mdx}* rats with anti-CD45RC antibody could be used to treat DMD by specifically acting on cells expressing CD45RC, hereby designated as CD45RC+ cells, and in particular acting on cells expressing high levels of CD45RC, hereby designed as CD45RC^{high} cells or CD45RC+^{high} cells.

Moreover, inventors showed that administration to Aire^{-/-} (KO) rats of an anti-CD45RC monoclonal antibody results in a strong depletion of CD45RC^{high} T lymphocytes, and to the removal of symptoms characteristics of APECED.

The present invention therefore relates to an anti-CD45RC antibody for use in the treatment of monogenic diseases, wherein genes involved in said monogenic diseases are:
- genes which are not associated with immune function but whose deficiency is associated with inflammation and/or immune reactions, such as genes deficient in the following diseases: Duchenne muscular dystrophy (DMD), cystic fibrosis, lysosomal diseases and alpha1-anti-trypsin deficiency; or
- genes involved in the immune system and whose deficiency generates inflammation and/or autoimmune reactions, such as genes deficient in the following diseases: T-cell primary immunodeficiencies such as IPEX (immunodysregulation polyendocrinopathy enteropathy X-linked syndrome), APECED (autoimmune polyendocrinopathy-candidiasis-ectodermal dystrophy), B cell primary immunodeficiencies, Muckle-Wells syndrome, mixed autoinflammatory and autoimmune syndrome, NLRP12-associated hereditary periodic fever syndrome, and tumor necrosis factor receptor 1 associated periodic syndrome.

In the sense of the invention, a "deficient gene" is a gene that is mutated or absent or non-functional, and as a consequence the encoded protein is missing, or is present in a tiny, non effective amount, or is under a mutated, inactive form.

The term "monogenic diseases" refers herein to diseases resulting from a mutation in a single gene selected among the following genes:
- genes which are not associated with immune function but whose deficiency is associated with inflammation and/or immune reactions, such as genes deficient in the following diseases: Duchenne muscular dystrophy (DMD), cystic fibrosis, lysosomal diseases and alpha1-anti-trypsin deficiency; and
- genes involved in the immune system and whose deficiency generates inflammation and/or autoimmune reactions, such as genes deficient in the following diseases: T-cell primary immunodeficiency such as IPEX (immunodysregulation polyendocrinopathy enteropathy X-linked syndrome), APECED (autoimmune polyendocrinopathy-candidiasis-ectodermal dystrophy), B cell primary immunodeficiencies, Muckle-Wells syndrome, mixed autoinflammatory and autoimmune syndrome, NLRP12-associated hereditary periodic fever syndrome, and tumor necrosis factor receptor 1 associated periodic syndrome.

As used herein, the terms "treat", "treating" or "treatment" refers to the administration of therapy to an individual in an attempt to reduce the frequency and/or severity of symptoms of a disease, defect, disorder, or adverse condition of a patient.

### Anti-CD45RC antibodies

As used herein, the term "CD45" refers to a transmembrane glycoprotein existing in different isoforms. These distinct isoforms of CD45 differ in their extracellular domain structures which arise from alternative splicing of 3 variable exons coding for part of the CD45 extracellular region. The various isoforms of CD45 have different extracellular domains, but have the same transmembrane and cytoplasmic segments having two homologous, highly conserved phosphatase domains of approximately 300 residues.

The term "CD45RC" refers to a 200-220 kD single chain type I membrane glycoprotein well known from the skilled man in the art. It is an exon 6 splice variant (exon C) of the tyrosine phosphatase CD45.

The CD45RC isoform is expressed at high levels on B cells, and subsets of CD8⁺ T cells, CD4⁺T cells and dendritic as well as Natural Killer cells. CD45 and its isoforms non-covalently associate with lymphocyte phosphatase-associated phosphoprotein (LPAP) on T and B lymphocytes.

CD45 has been reported to be associated with several other cell surface antigens, including CD1, CD2, CD3, and CD4. CD45 is involved in signaling lymphocytes activation.

By "antibody" or "immunoglobulin", is meant a molecule comprising at least one domain for binding to a given antigen, and a constant domain comprising an Fc fragment capable of binding to FcR receptors. The term antibody encompasses not only whole antibody molecules, but also antibody fragments as well as derivatives of antibodies and of antibody fragments. In most mammals, like humans and mice, an antibody consists of 4 polypeptide chains: 2 heavy chains and 2 light chains connected together through a variable number of disulfide bridges ensuring flexibility to the molecule. Each light chain consists of a constant domain (CL) and of a variable domain (VL); the heavy chains consists of a variable domain (VH) and of 3 or 4 constant domains (CH1 to CH3 or CH1 to CH4) according to the isotype of the antibody. In a few rare mammals, like camels and lamas, the antibodies only consist of two heavy chains, each heavy chain comprising a variable domain (VH) and a constant region. Moreover, the term antibody according to the invention encompasses bispecific antibody that has binding sites for two different antigens within a single antibody molecule.

As used herein "Fc region" includes the polypeptides comprising the constant region of an antibody excluding the first constant region immunoglobulin domain. Thus Fc refers to the last two constant region immunoglobulin domains of IgA, IgD, and IgG, and the last three constant region immunoglobulin domains of IgE and IgM, and the flexible hinge N-terminal to these domains. For IgA and IgM Fc may include the J chain. For IgG, Fc comprises immunoglobulin domains Cgamma2 and Cgamma3 (Cγ2 and Cγ3) and the hinge between Cgamma1 (Cγ1) and Cgamma2 (Cγ2).

Although the boundaries of the Fc region may vary, the human IgG heavy chain Fc region is usually defined to comprise residues C226 or P230 to its carboxyl-terminus, wherein the numbering is according to the EU index as in Kabat et al. (1991, NIH Publication 91-3242, National Technical Information Service, Springfield, Va.). The "EU index as set forth in Kabat" refers to the residue numbering of the human IgG1 EU antibody as described in Kabat et al. supra. Fc may refer to this region in isolation, or this region in the context of an antibody, antibody fragment, or Fc fusion protein.

"Functional fragment" or "antibody fragment" means an antibody conserving the antigen binding domain and therefore having the same antigenic specificity as the original antibody, such as fragments Fv, ScFv, Fab, F(ab')2, Fab', scFv-Fc or diabodies. In the sense of the invention, the Fc fragment of an antibody may be natural or may have been modified in various ways, provided that it comprises a functional domain for binding to FcR receptors (FcγR receptors for IgGs), and preferably a functional domain for binding to receptor FcRn. The modifications may include the deletion of certain portions of the Fc fragment, provided that the latter contains a functional domain for binding to receptors FcR (receptors FcγR for IgGs), and preferably a functional domain for binding to receptor FcRn. The modifications of the antibody or the Fc fragment of an antibody may also include various substitutions of amino acids able to affect the biological properties of the antibody, provided that the latter contains a functional domain for binding to receptors FcR, and preferably a functional domain for binding to receptor FcRn.

In particular, when the antibody is an IgG, it may comprise mutations intended to enhance the binding to receptor FcγRIII (CD16), as described in WO00/42072, WO2004/029207, WO2004/063351, WO2004/074455. Mutations permitting to enhance the binding to receptor FcRn and therefore the half-life *in vivo* may also be present, as described for example in WO00/42072, WO02/060919, WO2010/045193, or WO2010/106180. Other mutations, such as those permitting to reduce or increase the binding to the proteins of the complement and therefore the Complement Dependent Cytotoxicity (CDC) response, may be present or not (see WO99/51642, WO2004074455).

A "derivative" of an antibody means a binding protein formed of a support peptide and at least one CDR of the original antibody, preserving its ability to recognize specifically an antigen, such as in the present case CD45RC.

Variable domains are involved in recognition of the antigen, while constant domains are involved in biological, pharmacokinetic and effector properties of the antibody.

Unlike variable domains, for which the sequence strongly varies from one antibody to another, constant domains are characterized by an amino acid sequence very close from one antibody to the other, typical of the species and of the isotype, with optionally a few somatic mutations.

The Fc fragment naturally consists of the constant region of the heavy chain excluding domain CH1, i.e. of the lower boundary region and of the constant domains CH2 and CH3 or CH2 to CH4 (depending on the isotype).

Antibodies directed against the CD45RC surface marker can be obtained according to known methods by administering the appropriate antigen or epitope (such as the peptide of SEQ ID NO: 2) to a host animal selected, e.g., from rats, pigs, cows, horses, rabbits, goats, sheep, Camelidae (camel, dromedary, llama) and mice, among others. Various adjuvants known in the art can be used to enhance antibody production. Techniques for production and isolation include but are not limited to the hybridoma technique, the human B-cell hybridoma technique and the EBV-hybridoma technique. Alternatively, techniques described for the production of single chain antibodies (see U.S. Pat. No. 4,946,778) can be adapted to produce single chain antibodies against the CD45RC surface marker.

Examples of anti-CD45RC antibody have been described in WO 2016/016442 and the content of this patent application is specifically incorporated herein by reference in its entirety.

In a preferred embodiment, the anti-CD45RC antibody for use according to the invention is a monoclonal antibody, preferentially is an anti-human CD45RC monoclonal antibody.

By "monoclonal antibody" is meant an antibody molecule having an identical and unique antigen specificity. The antibody molecules may vary as regards their post-translational modifications, and notably as regards their glycosylation structures or their isoelectric point, but have all been encoded by the same heavy and light chain sequences and therefore have, before any post-translational modification, the same protein sequence. Certain differences in protein sequences, related to post-translational modifications (such as for example the cleavage of the C-terminal lysine of the heavy chain, deamidation of asparagine residues and/or isomerization of aspartate residues), may nevertheless exist between the various antibody molecules present in a monoclonal antibody composition.

Monoclonal antibodies directed against CD45RC, or CD45RC^{high} cell surface marker, are well known from the skilled man in the art such as the antibodies commercialized. Examples of antibodies which bind the CD45RC antigen that are contemplated by the invention include antibodies such as the mouse monoclonal antibodies OX-22 (anti-rat CD45RC), OX-32 (anti-rat CD45RC), MT2 (anti-human CD45RC), and RP1/12 (anti-human CD45RC) or a derivative thereof. Such monoclonal antibodies are well known from the skilled man in the art and are commercialized by several companies (Spickett et al. 1983).

Anti-CD45RC monoclonal antibody recognizes the product of exon C and specifically binds to the polypeptide sequence defined by SEQ ID NO: 2 and encoded by the nucleotide sequence SEQ ID NO: 1 (exon 6, isoform C) as listed in the following Table 1:

**Table 1. Nucleotide and polypeptide sequence of exon 6**

| Exon 6 (SEQ ID NO. 1) | Product of exon 6 (SEQ ID NO. 2) |
|---|---|
| atgtcccaggagagaggagtacagccagcacctttcctaca gacccagtttccccattgacaaccaccctcagccttgcaca ccacagctctgctgccttacctgcacgcacctccaacacca ccatcacagcgaacacctcag | CPRREEYSQHLSYRPSFPIDNHPQPCTPQLCCLTC THLQHHHHSEHL |

As used herein, the terms "CD45RC⁺ cell surface marker" or "CD45RC⁺ cell antigen" refer to an antigen of SEQ ID NO:2 expressed on the surface of a CD45RC⁺ cells (including T cells, B cells and natural killer (NK) cells) which can be targeted with an anti-CD45RC agent which binds thereto (such as an antibody or an aptamer). Exemplary CD45RC⁺ T cell surface markers include but are not limited to the CD45RC as previously described or other antigens that characterize said population of T cells. The CD45RC⁺ T cells surface marker of particular interest is preferentially expressed on CD45RC⁺ T cells compared to other non-CD45RC⁺ T cells of a mammal.

Then after raising antibodies directed against the CD45RC cell surface marker as above described, the skilled man in the art can easily select those that act on CD45RC^{high} cells, for example those that deplete CD45RC⁺ cells via antibody-dependent cell mediated cytotoxicity (ADCC), complement dependent cytotoxicity (CDC), inhibition of CD45RC⁺ cell proliferation or induction of CD45RC⁺ cell death (e.g. via apoptosis) after direct binding of the antibody.

As used herein, the terms "deplete" or "depleting" with respect to cells expressing CD45RC, refers to a measurable decrease in the number of cells in the subject. The reduction can be at least about 10%, e.g., at least about 20%, 30%, 40%, 50%, 60%, 70%, 80%, 90%, 95%, 96%, 97%, 98%, 99%, or more. In some embodiments, the term refers to a decrease in the number of CD45RC+ cells in a subject or in a sample to an amount below detectable limits. According to the present invention, the antibody specifically mediates depletion of the effector cells strongly expressing CD45RC, in particular those designed as CD45RC^{high} T_{eff}.

The antibody according to the invention is able to mediate depletion of any type of cells expressing CD45RC on a high or intermediary level.

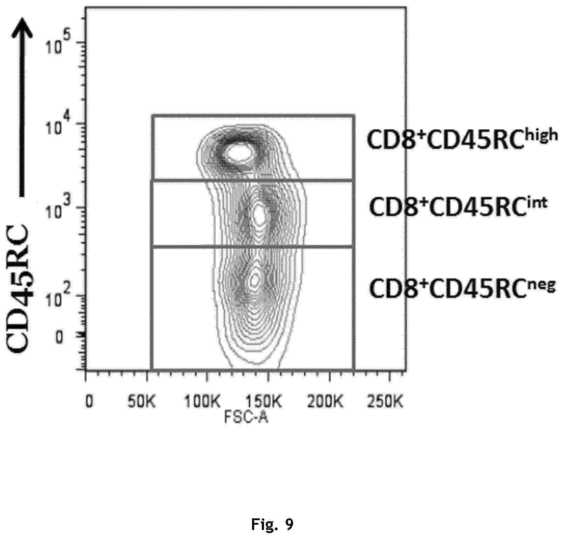

The relative level of expression of CD45RC is measured using cytofluorometry. Three types of cells can be distinguished: cells presenting a high, intermediary or negative level of CD45RC expression, as illustrated in Figure 9. Only cells presenting a high or intermediary level of CD45RC expression are designated as CD45RC+.

In a specific embodiment of the invention, the anti-CD45RC antibody for use according to the invention is an anti-CD45RC antibody that depletes T CD45RC^{high} cells.

The T CD45RC^{high} cells are T lymphocytes that express the CD45RC marker (CD45RC+) in high quantity, as defined above.

It is understood that said antibody that depletes T CD45RC+^{high} cells may be able to deplete other types of CD45RC+ cells, such as NK or B CD45RC+ cells.

In particular, said antibody depletes T CD45RC+^{high} cells by binding to CD45RC and transducing pro-apoptotic signals and/or by activating antibody-dependent cell-mediated cytotoxicity (ADCC), complement dependent cytotoxicity (CDC), or antibody-dependent phagocytosis.

The term "antibody-dependent cell-mediated cytotoxicity" or "ADCC" refers to a form of cytotoxicity in which secreted antibodies bound onto Fc receptors (FcRs) present on certain cytotoxic cells (e.g. NK cells, neutrophils, monocytes and macrophages) enable these cytotoxic effector cells to bind specifically to an antigen-bearing target cell and subsequently to kill the target cell. To assess ADCC activity of a molecule of interest, an in vitro ADCC assay, such as that described in US Patent No. 5,500,362 or 5,821,337 may be performed.

In some embodiments, the antibody of the present invention mediates complement dependant cytotoxicity.The term "complement dependent cytotoxicity" (CDC) refers to the lysis of a target cell in the presence of complement. Activation of the classical complement pathway is initiated by the binding of the first component of the complement system to antibodies which are bound to their cognate antigen. To assess complement activation, a CDC assay, e.g. as described in Gazzano-Santoro et al. (1997) may be performed. In a particular embodiment, the anti-CD45RC antibody may consist in an antibody directed against the CD45RC surface marker which is conjugated to a cytotoxic agent or a growth inhibitory agent.

In some embodiments, the antibody of the present invention mediates antibody-dependent phagocytosis. As used herein, the term "antibody-dependent phagocytosis" or "opsonisation" refers to the cell-mediated reaction wherein nonspecific cytotoxic cells that express FcγRs recognize bound antibody on a target cell and subsequently cause phagocytosis of the target cell.

### Preferred embodiments

In a particular embodiment, the anti-CDR45RC antibody according to the invention is a monoclonal antibody "MT2" or "RP1/12" or a derivative thereof. Such monoclonal antibodies are well known from the skilled man in the art and are commercialized by several companies.

The expression "a derivative of MT2" refers to an anti-CD45RC antibody which specifically binds to human CD45RC and which comprises the 6 CDRs of MT2.

In one embodiment, the derivative of MT2 is an antibody which comprises the VL chain and the VH chain of MT2.

In another embodiment, the derivative of MT2 is a chimeric antibody or humanized antibody, which comprises the variable domains of MT2.

The expression "a derivative of RP1/12" refers to an anti-CD45RC antibody which specifically binds to human CD45RC and which comprises the 6 CDRs of the monoclonal antibody RP1/12.

In one embodiment, the derivative of the monoclonal antibody RP 1/12 is an antibody which comprises the VL chain and the VH chain of the monoclonal antibody RP 1/12.

In a preferred embodiment, the antibody according to the invention is a chimeric antibody or a bispecific antibody or a humanized antibody or a fully human antibody or an antibody fragment or a derivative therefrom. Indeed, this gives the possibility of avoiding immune reactions of the patient against the administered antibodies.

By "chimeric" antibody, it is meant to designate an antibody which contains a natural variable region (light chain and heavy chain) derived from an antibody of a given species associated with constant regions of light chain and heavy chain of an antibody of a species heterologous to said given species. Advantageously, if the monoclonal antibody according to the invention is a chimeric monoclonal antibody, the latter comprises human constant regions. Starting from a non-human antibody, a chimeric antibody may be prepared by using genetic recombinant techniques well known to one skilled in the art. For example, the chimeric antibody may be produced by cloning for the heavy chain and the light chain a recombinant DNA including a promoter and a sequence coding for the variable region of the non-human antibody, and a sequence coding for the constant region of a human antibody. As for the methods for preparing chimeric antibodies, reference may for example be made to document Verhoeyn et al. 1988.

In some embodiments, the antibody of the present invention is a multispecific antibody comprising a first antigen binding site directed against CDR45RC and at least one second antigen binding site directed against an effector cell, said effector cell being able to mediate depletion of T CD45RC^{high} cells through direct binding, antibody-dependent cell-mediated cytotoxicity (ADCC), complement dependent cytotoxicity (CDC) and/or antibody dependent phagocytosis.

In said embodiments, the second antigen-binding site is used for recruiting a killing mechanism such as, for example, by binding an antigen on a human effector cell. In some embodiments, an effector cell is capable of inducing ADCC, such as a natural killer cell. For example, monocytes, macrophages, which express FcRs, are involved in specific killing of target cells and presenting antigens to other components of the immune system. In some embodiments, an effector cell may phagocytose a target antigen or target cell. The expression of a particular FcR on an effector cell may be regulated by humoral factors such as cytokines. An effector cell can phagocytose a target antigen or phagocytose or lyse a target cell. Suitable cytotoxic agents and second therapeutic agents are exemplified below, and include toxins (such as radiolabeled peptides), chemotherapeutic agents and prodrugs. In some embodiments, the second binding site binds to a Fc receptor as above defined. In some embodiments, the second binding site binds to a surface molecule of NK cells so that said cells can be activated. In some embodiments, the second binding site binds to NKp46.

Exemplary formats for the multispecific antibody molecules of the present invention include, but are not limited to (i) two antibodies cross-linked by chemical heteroconjugation, one with a specificity to a specific surface molecule of ILC and another with a specificity to a second antigen; (ii) a single antibody that comprises two different antigen-binding regions; (iii) a single-chain antibody that comprises two different antigen-binding regions, e.g., two scFvs linked in tandem by an extra peptide linker; (iv) a dual-variable-domain antibody (DVD-Ig), where each light chain and heavy chain contains two variable domains in tandem through a short peptide linkage; (v) a chemically-linked bispecific (Fab')2 fragment; (vi) a Tandab, which is a fusion of two single chain diabodies resulting in a tetravalent bispecific antibody that has two binding sites for each of the target antigens; (vii) a flexibody, which is a combination of scFvs with a diabody resulting in a multivalent molecule; (viii) a so called "dock and lock" molecule, based on the "dimerization and docking domain" in Protein Kinase A, which, when applied to Fabs, can yield a trivaient bispecific binding protein consisting of two identical Fab fragments linked to a different Fab fragment; (ix) a so-called Scorpion molecule, comprising, e.g., two scFvs fused to both termini of a human Fab-arm; and (x) a diabody. Another exemplary format for bispecific antibodies is IgG-like molecules with complementary CH3 domains to force heterodimerization. Such molecules can be prepared using known technologies, such as, e.g., those known as Triomab/Quadroma (Trion Pharma/Fresenius Biotech), Knob-into-Hole (Genentech), CrossMAb (Roche) and electrostatically-matched (Amgen), LUZ-Y (Genentech), Strand Exchange Engineered Domain body (SEEDbody)(EMD Serono), Biclonic (Merus) and DuoBody (Genmab A/S) technologies.

In some embodiments, a bispecific antibody is obtained or obtainable via a controlled Fab-arm exchange, typically using DuoBody® technology. *In vitro* methods for producing bispecific antibodies by controlled Fab-arm exchange have been described in WO2008119353 and WO 2011131746 (both by Genmab A/S).

In one exemplary method, described in WO 2008119353, a bispecific antibody is formed by "Fab-arm" or "half- molecule" exchange (swapping of a heavy chain and attached light chain) between two monospecific antibodies, both comprising IgG4-like CH3 regions, upon incubation under reducing conditions. The resulting product is a bispecific antibody having two Fab arms which may comprise different sequences.

In another exemplary method, described in WO 2011131746, bispecific antibodies are prepared by a method comprising the following steps, wherein at least one of the first and second antibodies is a human monoclonal antibody of the present invention:
a) providing a first antibody comprising an Fc region of an immunoglobulin, said Fc region comprising a first CH3 region;
b) providing a second antibody comprising an Fc region of an immunoglobulin, said Fc region comprising a second CH3 region;
   wherein the sequences of said first and second CH3 regions are different and are such that the heterodimeric interaction between said first and second CH3 regions is stronger than each of the homodimeric interactions of said first and second CH3 regions;
c) incubating said first antibody together with said second antibody under reducing conditions; and
d) obtaining said bispecific antibody, wherein the first antibody is a human monoclonal antibody of the present invention and the second antibody has a different binding specificity, or vice versa.

The reducing conditions may, for example, be provided by adding a reducing agent, e.g. selected from 2-mercaptoethylamine, dithiothreitol and tris(2-carboxyethyl)phosphine. Step d) may further comprise restoring the conditions to become non-reducing or less reducing, for example by removal of a reducing agent, e.g. by desalting. Preferably, the sequences of the first and second CH3 regions are different, comprising only a few, fairly conservative, asymmetrical mutations, such that the heterodimeric interaction between said first and second CH3 regions is stronger than each of the homodimeric interactions of said first and second CH3 regions. More details on these interactions and how they can be achieved are provided in WO 2011131746, which is hereby incorporated by reference in its entirety.

The following are exemplary embodiments of combinations of such assymetrical mutations, optionally wherein one or both Fc-regions are of the IgG1 isotype. In some embodiments, the first Fc region has an amino acid substitution at a position selected from the group consisting of: 366, 368, 370, 399, 405, 407 and 409, and the second Fc region has an amino acid substitution at a position selected from the group consisting of: 366, 368, 370, 399, 405, 407 and 409, and wherein the first and second Fc regions are not substituted in the same positions. In some embodiments, the first Fc region has an amino acid substitution at position 405, and said second Fc region has an amino acid substitution at a position selected from the group consisting of: 366, 368, 370, 399, 407 and 409, optionally 409. In some embodiments, the first Fc region has an amino acid substitution at position 409, and said second Fc region has an amino acid substitution at a position selected from the group consisting of: 366, 368, 370, 399, 405, and 407, optionally 405 or 368. In some embodiments, both the first and second Fc regions are of the IgG1 isotype, with the first Fc region having a Leu at position 405, and the second Fc region having an Arg at position 409. These point mutations, indicated according to Kabat numbering, are well known by the man skilled in the art, since they are disclosed in several patent applications, such as for example in WO 2016/091891.

By "humanized" antibody, it is meant to designate an antibody which contains CDR regions derived from an antibody of non-human origin, the other portions of the antibody molecule being derived from one (or from several) human antibodies. Further, certain of the residues of the backbone segments (called FR) may be modified for retaining the binding affinity (Jones et al. 1986; Verhoeyen et al.- 1988; Riechmann et al. 1988). The humanized antibodies according to the invention may be prepared by techniques known to one skilled in the art such as "CDR grafting", "resurfacing", SuperHumanization, "Human string content", "FR libraries", "Guided selection", "FR shuffling" and "Humaneering" techniques, as summarized in the review of Almagro et al. 2008.

By "human" or "fully human" antibody, is meant an antibody for which the whole sequence is of human origin, i.e. for which the coding sequences have been produced by recombination of human genes coding for antibodies. Indeed, it is now possible to produce transgenic animals (for ex. mice) which are capable, upon immunization, of producing a complete list of human antibodies in the absence of endogenous immunoglobulin production (see Jakobovits et al. 1993(a) and (b); Bruggermann et al.-1993; and Duchosal et al.-1992, U.S. patents 5,591,669, 5,598,369, 5,545,806, 5,545,807, 6,150,584). The human antibodies may also be obtained from phage display banks (Hoogenboom et al. 1991; Marks et al.-1991; Vaughan et al.-1996).

The antibodies may be of several isotypes, depending on the nature of their constant region: constant regions γ, α, µ, ε and δ respectively correspond to IgG, IgA, IgM, IgE and IgD immunoglobulins.

Advantageously, the monoclonal antibody according to the invention is of an IgG isotype. Indeed, this isotype shows an ability to generate ADCC activity in the largest number of individuals (humans). γ constant regions comprise several sub-types: γ1, γ2, γ3, these three types of constant regions having the particularity of binding the human complement, and γ4, thereby generating sub-isotypes IgG1, IgG2, IgG3, and IgG4. Advantageously, the monoclonal antibody according to the invention is of an isotype IgG1 or IgG3, preferably IgG1. The monoclonal antibody may be produced by a cell clone, a non-human transgenic animal or a transgenic plant, by technologies well known to one skilled in the art.

Fully human monoclonal antibodies directed against human CD45RC according to the invention can be generated using transgenic or transchromosomic mice carrying parts of the human immune system rather than the mouse system. These transgenic and transchromosomic mice include mice referred to herein as the HuMAb Mouse® and KM Mouse®, respectively, and are collectively referred to herein as "human Ig mice."

The HuMAb Mouse® (Medarex®, Inc.) contains human immunoglobulin gene miniloci that encode unrearranged human heavy (µ and γ) and κ light chain immunoglobulin sequences, together with targeted mutations that inactivate the endogenous µ and κ chain loci (see e.g., Lonberg et al. (1994a)). Accordingly, the mice exhibit reduced expression of mouse IgM or κ, and in response to immunization, the introduced human heavy and light chain transgenes undergo class switching and somatic mutation to generate high affinity human IgGK monoclonal antibodies (Lonberg et al. (1994b), Lonberg, N. and Huszar, D. (1995), and Harding and Lonberg (1995)). Preparation and use of the HuMAb Mouse®, and the genomic modifications carried by such mice, is further described in Taylor et al. (1992); Chen et al. (1993); Tuaillon et al. (1993); Choi et al. (1993); Tuaillon et al. (1994); Taylor et al. (1994); and Fishwild et al. (1996), the contents of all of which are hereby specifically incorporated by reference in their entirety. See further, U.S. Patent Nos. 5,545,806; 5,569,825; 5,625, 126; 5,633,425; 5,789,650; 5,877,397; 5,661,016; 5,814,318; 5,874,299; 5,770,429; and 5,545,807; PCT Publication Nos. WO 92/03918; WO 93/12227; WO 94/25585; WO 97/13852; WO 98/24884; WO 99/45962 and WO 01/14424, the contents of which are incorporated herein by reference in their entirety. Fully human antibodies of the invention can also be raised using a mouse that carries human immunoglobulin sequences on transgenes and transchromosomes, such as a mouse that carries a human heavy chain transgene and a human light chain transchromosome. This mouse is referred to herein as a "KM mouse" and is described in detail in PCT application WO 02/43478. A modified form of this mouse, which further comprises a homozygous disruption of the endogenous FcyRIIB receptor gene, is also described in PCT Publication WO 02/43478 and referred to herein as a "KM/FCGR2D mouse®." In addition, mice with either the HCo7 or HCol2 heavy chain transgenes or both can be used. Additional transgenic animal embodiments include the Xenomouse (Abgenix, Inc.,U.S. Patent Nos. 5,939,598; 6,075, 181; 6,114,598; 6,150,584 and 6,162,963). Further embodiments include "TC mice" (Tomizuka et al. (2000)) and cows carrying human heavy and light chain transchromosomes (Kuroiwa et al. (2002); WO 02/092812).

Still additional transgenic animal embodiments include the OmniRat™ (OMT, Inc., PCT WO 2008/151081). The contents of these patents and publications are specifically incorporated herein by reference in their entirety.

Moreover, antibody according to the invention can also be modified to enhance ADCC or Complement Dependent Cytotoxicity (CDC) response. Such antibody modifications are well known in the art.

For example, antibodies comprising a low fucose content are known to enhance ADCC response via the FcγRIII receptor (WO 2014/140322). Thus, the antibody according to the invention may comprise a low fucose content. By "fucose content", is meant the percentage of fucosylated forms within the N-glycans attached to the Asn297 residue of the Fc fragment of each heavy chain of each antibody. By "low fucose content" is meant a fucose content of less than or equal to 65%. Advantageously, the fucose content is less than or equal to 65%, preferably less than or equal to 60%, 55% or 50%, or even less than or equal to 45%, 40%, 35%, 30%, 25% or 20%. However, it is not necessary that the fucose content be zero, and it may for example be greater than or equal to 5%, 10%, 15% or 20%.

The antibody according to the invention may further comprises different types of glycosylation (N-glycans of the oligomannose or biantennary complex type, with a variable proportion of bisecting N-acetylglucosamine (GlcNAc) residues or galactose residues in the case of N-glycans of the biantennary complex type), provided that they have a low fucose content (see WO2007048077). For example, antibodies having slightly fucosylated N-glycans can be obtained as described in EP1176195A1, WO01/77181, WO2012/041768.

The N-glycans of the oligomannose type have reduced half-life *in vivo* as compared with N-glycans of the biantennary complex type. Consequently, advantageously, the antibodies according to the invention have on their N-glycosylation sites of the Fc fragment glycan structures of the biantennary complex type, with a low fucose content, as defined above.

In some embodiments, the antibody of the present invention is conjugated to a therapeutic moiety, i.e. a drug. The therapeutic moiety can be, e.g., a cytotoxin, a chemotherapeutic agent, a cytokine, an immunosuppressant, an immune stimulator, a lytic peptide, or a radioisotope. Such conjugates are referred to herein as an "antibody-drug conjugates" or "ADCs".

In some embodiments, the antibody of the present invention is conjugated to a cytotoxic moiety.

This cytotoxic moiety may, for example, be selected from the group consisting of taxol; cytochalasin B; gramicidin D; ethidium bromide; emetine; mitomycin; etoposide; tenoposide; vincristine; vinblastine; colchicin; doxorubicin; daunorubicin; dihydroxy anthracin dione; a tubulin- inhibitor such as maytansine or an analog or derivative thereof; an antimitotic agent such as monomethyl auristatin E or F or an analog or derivative thereof; dolastatin 10 or 15 or an analogue thereof; irinotecan or an analogue thereof; mitoxantrone; mithramycin; actinomycin D; 1-dehydrotestosterone; a glucocorticoid; procaine; tetracaine; lidocaine; propranolol; puromycin; calicheamicin or an analog or derivative thereof; an antimetabolite such as methotrexate, 6 mercaptopurine, 6 thioguanine, cytarabine, fludarabin, 5 fluorouracil, decarbazine, hydroxyurea, asparaginase, gemcitabine, or cladribine; an alkylating agent such as mechlorethamine, thioepa, chlorambucil, melphalan, carmustine (BSNU), lomustine (CCNU), cyclophosphamide, busulfan, dibromomannitol, streptozotocin, dacarbazine (DTIC), procarbazine, mitomycin C; a platinum derivative such as cisplatin or carboplatin; duocarmycin A, duocarmycin SA, rachelmycin (CC-1065), or an analog or derivative thereof; an antibiotic such as dactinomycin, bleomycin, daunorubicin, doxorubicin, idarubicin, mithramycin, mitomycin, mitoxantrone, plicamycin, anthramycin (AMC)); pyrrolo[2,l-c][l,4]-benzodiazepines (PDB); diphtheria toxin and related molecules such as diphtheria A chain and active fragments thereof and hybrid molecules, ricin toxin such as ricin A or a deglycosylated ricin A chain toxin, cholera toxin, a Shiga-like toxin such as SLT I, SLT II, SLT IIV, LT toxin, C3 toxin, Shiga toxin, pertussis toxin, tetanus toxin, soybean Bowman-Birk protease inhibitor, *Pseudomonas* exotoxin, alorin, saporin, modeccin, gelanin, abrin A chain, modeccin A chain, alpha-sarcin, Aleurites fordii proteins, dianthin proteins, Phytolacca americana proteins such as PAPI, PAPII, and PAP-S, momordica charantia inhibitor, curcin, crotin, sapaonaria officinalis inhibitor, gelonin, mitogellin, restrictocin, phenomycin, and enomycin toxins; ribonuclease (RNase); DNase I, Staphylococcal enterotoxin A; pokeweed antiviral protein; diphtherin toxin; and *Pseudomonas* endotoxin.

In some embodiments, the antibody of the present invention is conjugated to an auristatin or a peptide analog, derivative or prodrug thereof. Auristatins have been shown to interfere with microtubule dynamics, GTP hydrolysis and nuclear and cellular division (Woyke et al (2001) Antimicrob. Agents and Chemother. 45(12): 3580-3584) and have anti-cancer (US5663149) and antifungal activity (Pettit et al., (1998) Antimicrob. Agents and Chemother. 42: 2961-2965. For example, auristatin E can be reacted with para-acetyl benzoic acid or benzoylvaleric acid to produce AEB and AEVB, respectively. Other typical auristatin derivatives include AFP, MMAF (monomethyl auristatin F), and MMAE (monomethyl auristatin E). Suitable auristatins and auristatin analogs, derivatives and prodrugs, as well as suitable linkers for conjugation of auristatins to Antibodies, are described in, e.g., U.S. Patent Nos. 5,635,483, 5,780,588 and 6,214,345 and in International patent application publications WO02088172, WO2004010957, WO2005081711, WO2005084390, WO2006132670, WO03026577, WO200700860, WO207011968 and WO205082023.

In some embodiments, the antibody of the present invention is conjugated to pyrrolo[2,1-c][1,4]- benzodiazepine (PDB) or an analog, derivative or prodrug thereof. Suitable PDBs and PDB derivatives, and related technologies are described in the art.

In some embodiments, the antibody of the present invention is conjugated to a cytotoxic moiety selected from the group consisting of an anthracycline, maytansine, calicheamicin, duocarmycin, rachelmycin (CC-1065), dolastatin 10, dolastatin 15, irinotecan, monomethyl auristatin E, monomethyl auristatin F, a PDB, or an analog, derivative, or prodrug of any thereof.

In some embodiments, the antibody of the present invention is conjugated to an anthracycline or an analog, derivative or prodrug thereof. In some embodiments, the antibody is conjugated to maytansine or an analog, derivative or prodrug thereof. In some embodiments, the antibody is conjugated to calicheamicin or an analog, derivative or prodrug thereof. In some embodiments, the antibody is conjugated to duocarmycin or an analog, derivative or prodrug thereof. In some embodiments, the antibody is conjugated to rachelmycin (CC-1065) or an analog, derivative or prodrug thereof. In some embodiments, the antibody is conjugated to dolastatin 10 or an analog, derivative or prodrug thereof. In some embodiments, the antibody is conjugated to dolastatin 15 or an analog, derivative or prodrug thereof. In some embodiments, the antibody is conjugated to monomethyl auristatin E or an analog, derivative or prodrug thereof. In some embodiments, the antibody is conjugated to monomethyl auristatin F or an analog, derivative or prodrug thereof. In some embodiments, the antibody is conjugated to pyrrolo[2,l-c][l,4]-benzodiazepine or an analog, derivative or prodrug thereof. In some embodiments, the antibody is conjugated to irinotecan or an analog, derivative or prodrug thereof.

Techniques for conjugating molecule to antibodies, are well-known in the art (See, e.g., Arnon et al., "Monoclonal Antibodies For Immunotargeting Of Drugs In Cancer Therapy," in Monoclonal Antibodies And Cancer Therapy (Reisfeld et al. eds., Alan R. Liss, Inc., 1985); See also, e.g., PCT publication WO 89/12624.) Typically, the nucleic acid molecule is covalently attached to lysines or cysteines on the antibody, through N-hydroxysuccinimide ester or maleimide functionality respectively. Methods of conjugation using engineered cysteines or incorporation of unnatural amino acids have been reported to improve the homogeneity of the conjugate. Junutula et al. (2008) developed cysteine-based site-specific conjugation called "THIOMABs" (TDCs) that are claimed to display an improved therapeutic index as compared to conventional conjugation methods. Conjugation to unnatural amino acids that have been incorporated into the antibody is also being explored for ADCs; however, the generality of this approach is yet to be established. In particular the one skilled in the art can also envisage Fc-containing polypeptide engineered with an acyl donor glutamine-containing tag (e.g., Gin-containing peptide tags or Q- tags) or an endogenous glutamine that are made reactive by polypeptide engineering (e.g., via amino acid deletion, insertion, substitution, or mutation on the polypeptide). Then a transglutaminase, can covalently crosslink with an amine donor agent (e.g., a small molecule comprising or attached to a reactive amine) to form a stable and homogenous population of an engineered Fc-containing polypeptide conjugate with the amine donor agent being site- specifically conjugated to the Fc-containing polypeptide through the acyl donor glutamine-containing tag or the accessible/exposed/reactive endogenous glutamine (WO 2012059882).

In one embodiment, the anti-CD45RC antibody for use according to the invention is used in combination with a pharmaceutically acceptable carrier or excipient or vehicle.

The term "pharmaceutically" or "pharmaceutically acceptable" refers to molecular entities and compositions that do not produce an adverse, allergic or other untoward reaction when administered to a mammal, especially a human, as appropriate. A pharmaceutically acceptable carrier or excipient or vehicle refers to a non-toxic solid, semi-solid or liquid filler, diluent, encapsulating material or formulation auxiliary of any type. The form of the pharmaceutical compositions, the route of administration, the dosage and the regimen naturally depend upon the condition to be treated, the severity of the illness, the age, weight, and sex of the patient, etc. The pharmaceutical compositions of the invention can be formulated for a topical, oral, parenteral, intranasal, intravenous, intramuscular, subcutaneous administration and the like.

In a preferred embodiment, the anti-CD45RC antibody for use according to the invention is an anti-CD45RC monoclonal antibody selected from the group consisting of MT2 or RP1/12 or a derivative thereof and a pharmaceutically acceptable carrier or excipient or vehicle.

Therefore, an antibody of the invention may be combined with pharmaceutically acceptable excipients carrier or vehicle, and optionally sustained-release matrices, such as biodegradable polymers, to form therapeutic compositions.

Preferably, the pharmaceutical compositions contain vehicles which are pharmaceutically acceptable for a formulation capable of being injected. These may be in particular isotonic, sterile, saline solutions (monosodium or disodium phosphate, sodium, potassium, calcium or magnesium chloride and the like or mixtures of such salts), or dry, especially freeze-dried compositions which upon addition, depending on the case, of sterilized water or physiological saline, permit the constitution of injectable solutions. The doses used for the administration can be adapted as a function of various parameters, and in particular as a function of the mode of administration used, of the relevant pathology, or alternatively of the desired duration of treatment. To prepare pharmaceutical compositions, an effective amount of the antibody may be dissolved or dispersed in a pharmaceutically acceptable carrier or aqueous medium. The pharmaceutical forms suitable for injectable use include sterile aqueous solutions or dispersions; formulations including sesame oil, peanut oil or aqueous propylene glycol; and sterile powders for the extemporaneous preparation of sterile injectable solutions or dispersions. In all cases, the form must be sterile and must be fluid to the extent that easy syringability exists. It must be stable under the conditions of manufacture and storage and must be preserved against the contaminating action of microorganisms, such as bacteria and fungi.

Dosages to be administered depend on individual needs, on the desired effect and the chosen route of administration. It is understood that the dosage administered will be dependent upon the age, sex, health, and weight of the recipient, concurrent treatment, if any, frequency of treatment, and the nature of the effect desired. The total dose required for each treatment may be administered by multiple doses or in a single dose.

The doses used for the administration can be adapted as a function of various parameters, and in particular as a function of the mode of administration used, of the relevant pathology, or alternatively of the desired duration of treatment. For example, it is well within the skill of the art to start doses of the antibodies at levels lower than those required to achieve the desired therapeutic effect and to gradually increase the dosage until the desired effect is achieved. However, the daily dosage of the antibodies may be varied over a wide range from 0.01 to 1,000 mg per adult per day. Preferably, the compositions contain 0.01, 0.05, 0.1, 0.5, 1.0, 2.5, 5.0, 10.0, 15.0, 25.0, 50.0, 100, 250 and 500 mg of the active ingredient for the symptomatic adjustment of the dosage to the subject to be treated. A medicament typically contains from about 0.01 mg to about 500 mg of the active ingredient, preferably from 1 mg to about 100 mg of the active ingredient. A therapeutically effective amount of the drug is ordinarily supplied at a dosage level from 0.0002 mg/kg to about 20 mg/kg of body weight per day, especially from about 0.001 mg/kg to 10 mg/kg of body weight per day.

By "therapeutically effective amount" is meant an amount sufficient to achieve a concentration of antibodies which is capable of preventing, treating or slowing down the monogenic disease to be treated. Such concentrations can be routinely determined by those of skilled in the art.

### Use of the anti-CD45RC antibody in combination with other therapies

In a first embodiment, the anti-CD45RC antibody for use according to the invention is used alone.

In a second embodiment of the invention, the anti-CD45RC antibody is used in combination with an immunosuppressive and/or an anti-inflammatory drug selected from corticoids, such as glucocorticoids selected from dexamethasone, betamethasone, betamethasone-17-valerate, triamcinolone, triamcinolone acetonide, fluocinolone acetonide, fluocinonide, cortisone, hydrocortisone, prednisone, methylprednisolone, desonide, budesonide and deflazacort, and is preferentially used in combination with prednisolone or deflazacort.

The term "immunosuppressive and/or anti-inflammatory" relates to a class of drugs that suppress, or reduce, the strength of immune response to treat autoimmune disorders. The use of immunosuppressive drug according to the invention, along with the anti-CD45RC antibody according to the invention, would therefore help to reduce the impact of the immune response in the subject. Advantageously, immunosuppressive drug according to the invention is selected from corticoids such as glucocorticoids. Preferentially, glucocorticoids according to the invention are selected from dexamethasone, betamethasone, betamethasone-17-valerate, triamcinolone, triamcinolone acetonide, fluocinolone acetonide, fluocinonide, cortisone, hydrocortisone, prednisone, methylprednisolone, desonide, budesonide and deflazacort, preferentially prednisolone or deflazacort.

In a third embodiment of the invention, the anti-CD45RC antibody is used in combination with gene therapy or cell therapy. Advantageously, said gene therapy or cell therapy is used before or after the use of said anti-CD45RC antibody, preferentially before the use of said anti-CD45RC antibody.

In another embodiment of the invention, the anti-CD45RC antibody is used both in combination with an immunosuppressive and/or anti-inflammatory drug, and with gene therapy or cell therapy.

### Specific therapeutic uses of anti-CD45RC antibodies

In a first embodiment, the anti-CD45RC antibody for use according to the invention is used to treat monogenic diseases selected from DMD, cystic fibrosis and lysosomal storage diseases, this monogenic disease being preferably DMD.

In a second embodiment, the anti-CD45RC antibody for use according to the invention is used to treat monogenic diseases selected from T-cell primary immunodeficiencies such as IPEX (immunodysregulation polyendocrinopathy enteropathy X-linked syndrome), APECED (autoimmune polyendocrinopathy-candidiasis-ectodermal dystrophy), B cell primary immunodeficiencies, Muckle-Wells syndrome, mixed autoinflammatory and autoimmune syndrome, NLRP12-associated hereditary periodic fever syndrome, and tumor necrosis factor receptor 1 associated periodic syndrome, this monogenic disease being preferably APECED.

Another object of the present invention is a method of treatment of a monogenic disease selected from diseases involving:
- genes not associated with immune function but whose deficiency is associated with inflammation and/or immune reactions, such as genes deficient in the following diseases: Duchenne muscular dystrophy (DMD), cystic fibrosis, lysosomal diseases and alpha1-anti-trypsin deficiency; or
- genes involved in the immune system and whose deficiency generates inflammation and/or autoimmune reactions, such as genes deficient in the following diseases: T-cell primary immunodeficiencies including IPEX (immunodysregulation polyendocrinopathy enteropathy X-linked syndrome), APECED (autoimmune polyendocrinopathy-candidiasis-ectodermal dystrophy), B cell primary immunodeficiencies, Muckle-Wells syndrome, mixed autoinflammatory and autoimmune syndrome, NLRP12-associated hereditary periodic fever syndrome, tumor necrosis factor receptor 1 associated periodic syndrome,
comprising the step of administering an anti-CD45RC antibody or a composition comprising an anti-CD45RC antibody to a subject in need thereof.

Optionally, said administration of an anti-CD45RC antibody or a composition comprising an anti-CD45RC antibody to a subject is combined with the administration to the same subject of an immunosuppressive and/or an anti-inflammatory drug, and/or is combined with gene therapy or cell therapy.

In a preferred embodiment, the method of treatment of DMD comprises the step of administering to a subject in need thereof an anti-CD45RC antibody, or a composition comprising an anti-CD45RC antibody according to the invention and an immunosuppressive and/or anti-inflammatory drug selected from corticoids such as glucocorticoids selected from dexamethasone, betamethasone, betamethasone-17-valerate, triamcinolone, triamcinolone acetonide, fluocinolone acetonide, fluocinonide, cortisone, hydrocortisone, prednisone, methylprednisolone, desonide, budesonide and deflazacort, preferentially prednisolone or deflazacort .

In another embodiment, the method of treatment of DMD according to the invention comprises the step of administering to a subject in need thereof an anti-CD45RC antibody or a composition comprising an anti-CD45RC antibody and an immunosuppressive and/or anti-inflammatory drug according to the invention, before or after gene therapy, preferentially after gene therapy.

Another object of the present invention is a pharmaceutical composition comprising an anti-CD45RC antibody as defined previously, and an immunosuppressive and/or anti-inflammatory drug selected from corticoids such as glucocorticoids selected from dexamethasone, betamethasone, betamethasone-17-valerate, triamcinolone, triamcinolone acetonide, fluocinolone acetonide, fluocinonide, cortisone, hydrocortisone, prednisone, methylprednisolone, desonide, budesonide and deflazacort, preferentially prednisolone or deflazacort.

Another object of the present invention relates to a product containing an anti-CD45RC antibody or a composition comprising an anti-CD45RC antibody according to the invention, and an immunosuppressive and/or anti-inflammatory drug as described above, and/or an adeno-associated virus or lentivirus containing at least one mini-dystrophin gene, as a combined preparation for simultaneous, separate or sequential use in the treatment of monogenic diseases, such as DMD.

The term « mini-dystrophin gene » refers herein to 6- to 8-kb synthetic mini-dystrophin genes such as the mini-dystrophin genes described by Odom et al. 2011, Clemens et al., 1995, Harper et al., 2002 and Lai et al., 2009.

### LEGEND OF THE FIGURES

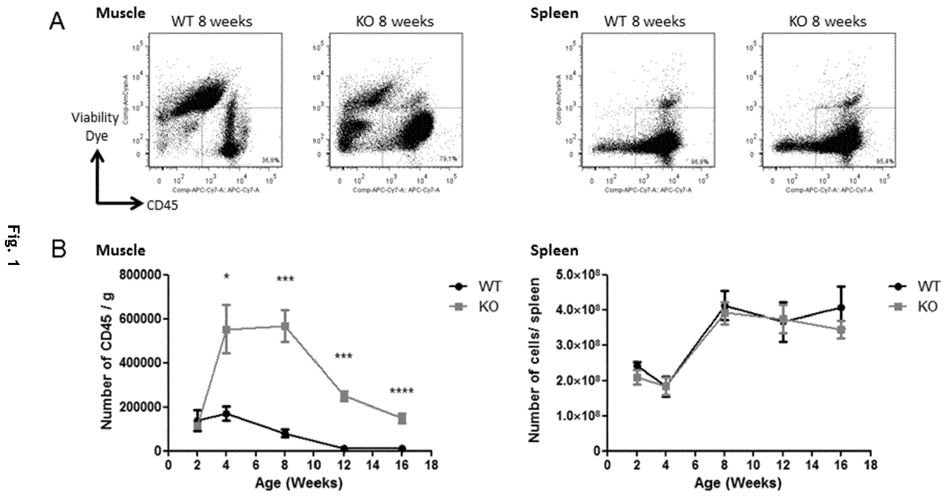

**Figure 1****. Number of leukocytes in muscle and spleen of *Dmd^{mdx}* rats.** Hind limb skeletal muscles and spleen were harvested from littermate wild-type (WT) or *Dmd^{mdx}* (KO) rats at the indicated time points of age. Muscle and spleen were digested with collagenase and mononuclear cells were isolated using a density gradient. A) Representative dot-plot analysis of mononuclear cells muscle (left panel) and spleen (right panel) from animals at 8 weeks of age stained with a viability dye and a pan anti-leukocyte CD45 monoclonal antibody (OX1). B) Number of CD45⁺ cells by gram of muscle tissue or by total spleen at different time points. WT, n=X. KO; n= Y. * p< 0.05, *** p< 0.001, and **** p< 0.0001.

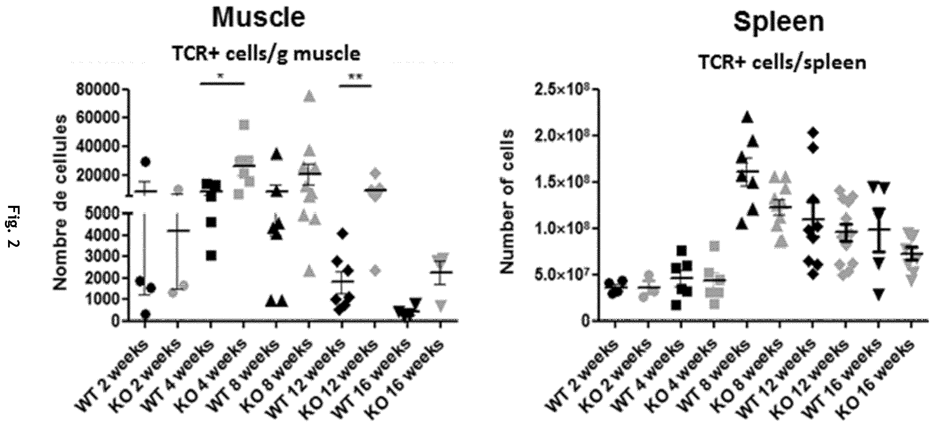

**Figure 2****. Number of TCR⁺ cells in muscle and spleen of littermate WT and *Dmd^{mdx}* rats.** PBMCs from hind limbs (left panel) and spleen (right panel) were obtained as explained in the legend of figure 1. The number of TCR⁺ cells was determined by staining PBMCs with an anti-TCRab monoclonal antibody (clone R7/3) and calculating the number of TCR⁺ cells among viable CD45R⁺ cells. *p< 0.05, **p<0.01.

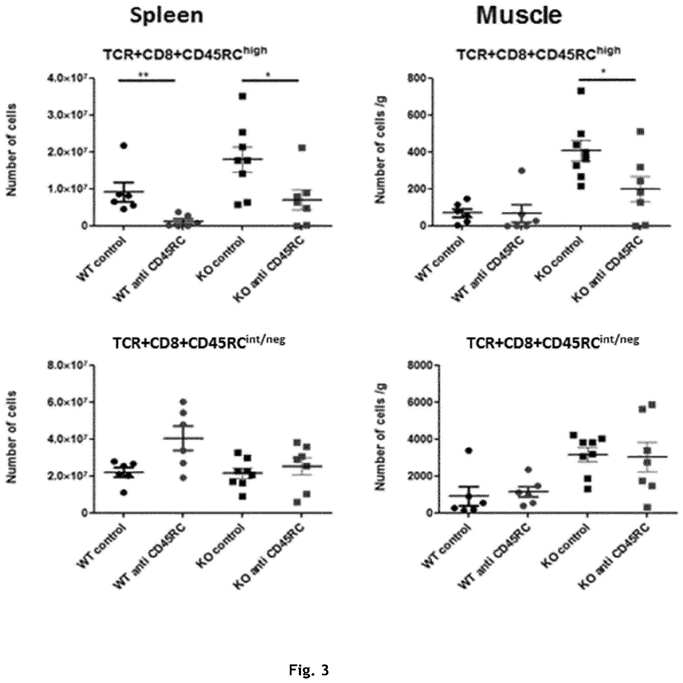

**Figure 3****. Numbers of T CD8+ CD45RC^{high} and CD45RC^{int/neg} cells after treatment using an anti-CD45RC monoclonal antibody.** Control littermate wild-type (WT) or *Dmd^{mdx}* (KO) rats received from week 2 of age intraperitoneal injections of the mouse anti-rat CD45RC monoclonal antibody up to week 12 when the animals were sacrificed and muscle and spleen mononuclear cells were isolated and analyzed for the presence of TCR⁺ CD8⁺ cells expressing CD45RC at either high (CD45RC^{high}) or int/negative levels (CD45RC^{int/neg}). Each point represents a single animal. *p< 0.05 and ***p< 0.001.

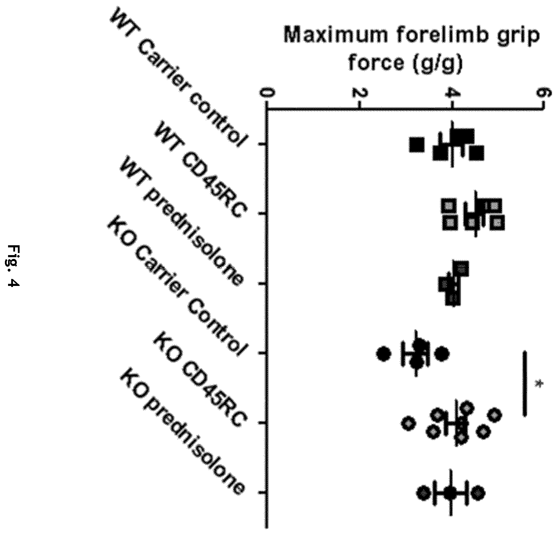

**Figure 4****. Muscle strength in *Dmd^{mdx}* rats after treatment with an anti-CD45RC monoclonal antibody.** Wild type (WT) or *Dmd^{mdx}* (KO) rats received intraperitoneal injections of the mouse anti-rat CD45RC or prednisolone from week 2 of age up to week 12 when the muscle strength was analyzed using a grip test. Each point represents a single animal. *p< 0.05.

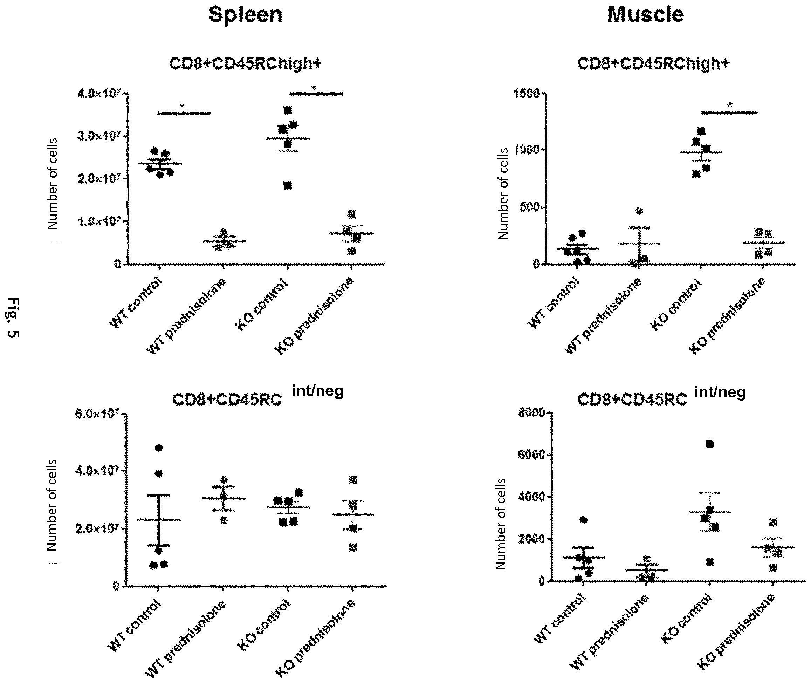

**Figure 5****. Numbers of T CD8+ CD45RC^{high} and CD45RC^{int/neg} cells after treatment using prednisolone.** Control littermate wild-type (WT) or *Dmd^{mdx}* (KO) rats received from week 2 of age intraperitoneal injections of prednisolone up to week 12 when the animals were sacrificed and muscle and spleen mononuclear cells were isolated and analyzed for the presence of TCR⁺ CD8⁺ cells expressing CD45RC at either high (CD45RC^{high}) or int/negative levels (CD45RC^{int/neg}). Each point represents a single animal. * p< 0.05.

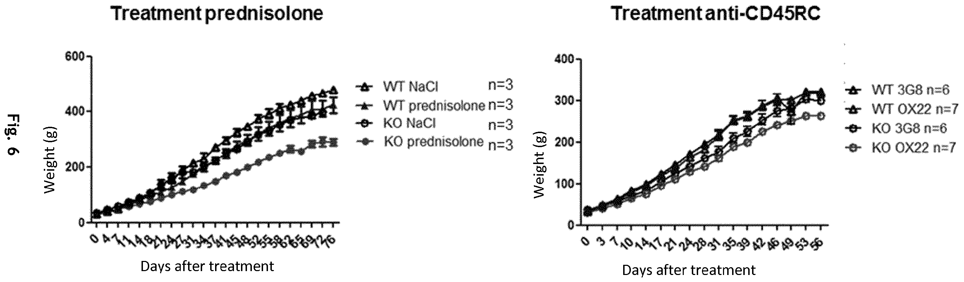

**Figure 6****. Weight of wild-type or *Dmd^{mdx}* rats during treatment using prednisolone or anti-CD45RC monoclonal antibody.** Control littermate wild-type (WT) or *Dmd^{mdx}* (KO) rats received intraperitoneal injections of prednisolone or anti-CD45RC monoclonal antibody (OX22) from week 2 up to week 12 of age and weight was recorded at the indicated time points.

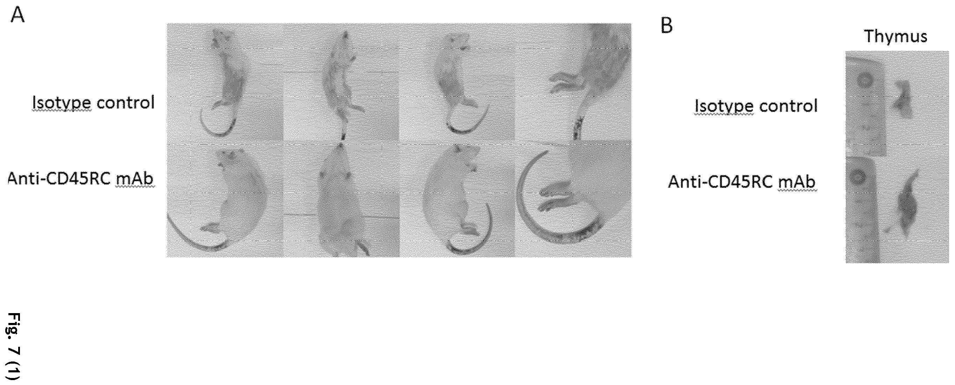

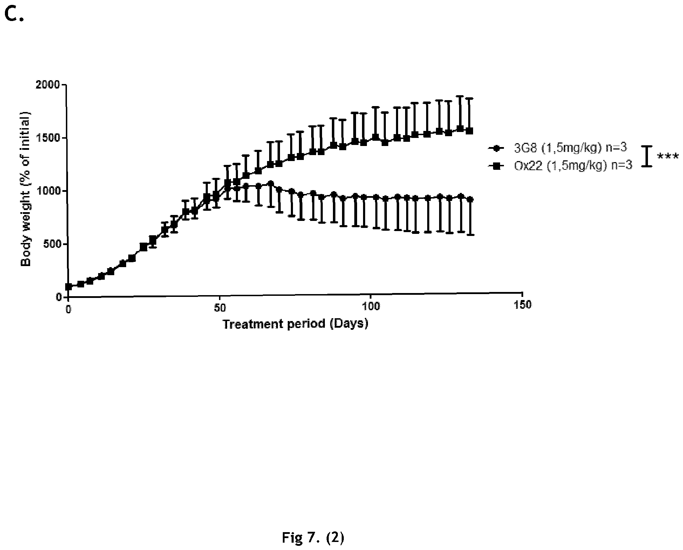

**Figure 7****. Anti-CD45RC mAb treated *Aire*^{*-*/*-*} rats showed reduced autoimmune symptoms.** *Aire*^{*-*/*-*} rats received from week 2 of age intraperitoneal injections of the mouse anti-rat CD45RC MAb (clone OX22, 1.5 mg/kg twice per week) up to week 20 when the animals were sacrificed
   **A.** Picture showing visual aspect of 20 weeks old *Aire*^{*-*/*-*} rats treated with isotype control (top row) or anti-CD45RC mAb (bottom row).
   **B.** Picture showing the size of the thymus from anti-CD45RC mAb or isotype control *Aire*^{*-*/-} treated rats
   **C.** Weights of anti-CD45RC mAb or isotype control *Aire*^{*-*/*-*} littermate SPD rats were measured every week during 20 weeks following birth. Results are shown as the % of initial body weight starting at week 2 after birth (mean 31,3 g) ± SEM (n=3).

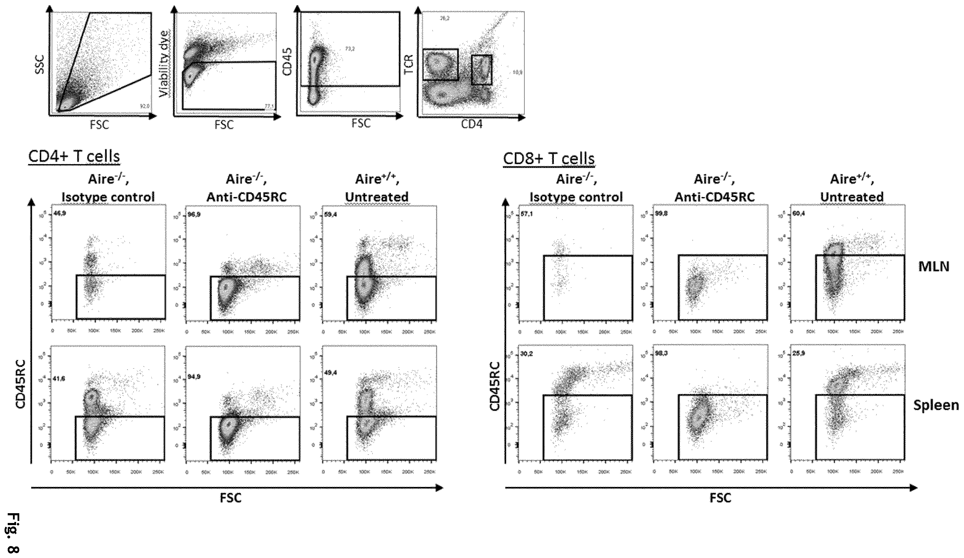

**Figure 8****. Anti-CD45RC mAb efficiently depleted CD45RC^{high} T cells in spleen and MLN.** Representative dot-plot analysis of mononuclear cells (CD4+ and CD8+ T cells) from MLN and spleen from *Aire*^{*-*/*-*} treated with anti-CD45RC or isotype control Mabs or WT untreated animals at 20 weeks of age and treated from week 2 stained with a viability dye and a pan anti-leukocyte CD45 MAb (OX1), CD3, TCR, CD4 and CD45RC MAb.
**Figure 9****.** Rat CD8+ T cells were labeled with the anti-CD45RC Mab OX22 and the different levels of CD45RC expression allow the definition of 3 populations among CD8+ cells: CD45RC^{high}, CD45RC^{int} and CD45RC^{neg}.

### EXAMPLES

### Materials and methods related to examples 1 to 4

### Preparation of muscle and spleen single-cell suspensions

Muscles of both hind limbs from WT or *Dmd^{mdx}* rats were excised and weighed. Muscles were minced and placed in gentle MACS C tubes with collagenase D in the presence of FCS 2% 1mM EDTA. Two runs of 30 min each in a gentle MACS dissociator were performed with new collagenase added between each run. Cells were suspended in 30 mL of PBS FCS 2% 1 mM EDTA, were then applied to 15 mL of Hystopaque and centrifuged at 1000g for 30 minutes without a break. Mononuclear cells were collected from the Hystopaque and PBS interface, washed and suspended in PBS FCS 2% 1mM EDTA. Spleen was harvested, perfused with PBS and digested by collagenase D for 15 min at 37°C, cells were suspended in PBS FCS 2% 1mM EDTA and mononuclear cells were recovered as explained above.

### Flow cytometry analysis

Mononuclear cells were stained with antibodies against the following antigens: CD45 (clone OX-1), T-cell receptor (TCR; clone R7/3), CD45RC (clone OX22), CD8 (clone OX8) and CD4 (W3/25) as well as with viability dyes eFluor506 or eFluor450, all from eBiosciences. Analysis was performed on a BD FACS Verse with FACSuite Software version 1.0.6. Post-acquisition analysis was performed with FlowJo software.

### Treatment with anti-CD45RC or prednisolone

WT or *Dmd^{mdx}* (KO) rats received intraperitoneal injections of a mouse anti-rat CD45RC monoclonal antibody (clone OX22, 2 mg/kg, every 3.5 days) from week 2 of age up to week 12. Prednisolone was administered by intraperitoneal injections (0.5 mg/kg, 5 days a week) from week 2 of age up to week 12. At week 12 of age treated rats were analyzed for muscle strength using a grip test.

### Grip test

Rats were placed with their forepaws on a grid and were gently pulled backward until they released their grip. A grip meter (Bio-GT3, BIOSEB, France), attached to a force transducer, measured the peak force generated.

### Example 1: Analysis of total leukocytes and T cells in Dmd^{mdx} rats

Leukocytes in the muscle and spleen of *Dmd^{mdx}* rats were analyzed by flow cytometry (Fig 1). Total leukocytes in the muscle of littermate wild type (WT) and *Dmd^{mdx}* rats were comparable at 2 weeks of age but at 4 weeks *Dmd^{mdx}* rats showed a sharp increase that was maintained until week 8 and then decreased at weeks 12 and 14 but that were still significantly higher than littermate WT rats. Spleen leukocyte numbers were comparable between WT and *Dmd^{mdx}* rats at all-time points analyzed.

Analysis of the number of TCR⁺ cells in muscle and spleen of littermate WT and *Dmd^{mdx}* rats showed a significant increase in the muscle of *Dmd^{mdx}* rats at 4 and 12 weeks of age with an increased tendency at week 8 and 16 weeks (Fig. 2). The numbers of TCR⁺ cells in spleen did not show differences between WT and *Dmd^{mdx}* rats at any time point.

### Example 2: Treatment with anti-CD45RC monoclonal antibody depletes CD45RC^{high} T cells

Administration of a mouse anti rat-CD45RC monoclonal antibody from week 2 of age resulted in partial depletion of T CD8⁺ CD45RC^{high} cells analyzed at 12 weeks of age in muscle of *Dmd^{mdx}* rats and in spleen of both littermate WT and *Dmd^{mdx}* rats (Fig. 3). T CD8⁺ or CD4⁺ CD45RC^{int/neg}, including all CD8⁺ and CD4⁺ T_{regs}, were not modified in the spleen or muscle of neither WT nor *Dmd^{mdx}* rats (Fig. 3). All other major leukocyte populations (macrophages, B cells and NK cells) were unchanged (data not shown).

### Example 3: Treatment with anti-CD45RC monoclonal antibody improves muscle strength in Dmd^{mdx} rats

To examine whether muscle function was ameliorated by the anti-CD45RC monoclonal antibody treatment, muscle strength was analyzed by a grip test of forelimbs in WT and *Dmd^{mdx}* rats at 12 weeks of age after initiation of treatment at 2 weeks of age. A significant decrease in forelimb grip strength indicated a generalized alteration in the whole body muscular performance. As previously described (Larcher et al., 2014) a 30% weaker force was exerted by *Dmd^{mdx}* rats compared to WT littermates. After administration of the anti-CD45RC monoclonal antibody, muscle strength in *Dmd^{mdx}* rats vs. untreated *Dmd^{mdx}* rats was significantly increased and was indistinguishable from wild-type littermate controls (Fig. 4).

### Example 4: Treatment with prednisolone improved skeletal muscle strength

Since corticoids are standard treatment in DMD patients (Alman, 2005), the effect of prednisolone was analyzed in the muscle strength of *Dmd^{mdx}* rats.

Treatment of *Dmd^{mdx}* rats with prednisolone, since 2 weeks of age, increased muscle strength at 12 weeks to levels identical to those of WT or anti-CD45RC-treated rats **(****Fig. 4****).**

Interestingly, prednisolone-treated rats also showed a specific decrease of CD8⁺CD45RC^{hilh} cells in both muscle and spleen of *Dmd^{mdx}* rats and in spleen of WT rats whereas CD8⁺CD45RC^{int/neg} cells were maintained **(****Fig. 5****).** Similarly, CD4⁺CD45RC^{high} cells were numerically reduced in both muscle and spleen of *Dmd^{mdx}* rats and in spleen of WT rats whereas CD8⁺CD45RC^{int/neg} cells were maintained **(data not shown).** Prednisolone-treated *Dmd^{mdx}* rats showed severe reduction in animal growth whereas anti-CD45RC monoclonal antibody-treated rats did not **(****Fig. 6****).**

### Example 5: Treatment of APECED disease

### Materials and methods

### Cell isolation

Spleen and lymph nodes were digested by collagenase D for 30 minutes at 37°C, the reaction was stopped by adding 0.01mM EDTA. Cells from blood and bone marrow were also isolated and red blood cells were lysed using a lysis solution (8,29g NH4Cl, 1g KHCO3, 37,2mg EDTA qsp 1L deionised water pH 7,2-7,4).

### Antibodies and flow cytometry

Cellular phenotype was analyzed using monoclonal antibodies from BD pharmigen: against TCRαβ (R73); CD25 (Ox39); CD4 (Ox35); CD45 (Ox1). Abs against CD45RC (Ox22) and CD8 (Ox8) produced in our lab were used. Antibodies were used to stain cells and fluorescence was measured with a FACSCanto II flow cytometer (BD Bioscience) and FlowJo software was used to analyze data. Cells were first gated on their morphology and then dead cells were excluded by staining with fixable viability dye efluor 506 (Ebioscience).

### Treatment with anti-CD45RC or isotype control

*Aire*^{*-*/*-*} (KO) rats received intraperitoneal injections of a mouse anti-rat CD45RC monoclonal antibody (clone OX22, 1,5 mg/kg, every 3.5 days) from week 2 of age up to week 20. Isotype control was administered similarly. The rats were weighted twice a week from the start of the treatment until the day of sacrifice. At week 20 of age, treated rats were sacrificed and analyzed.

### Results

*Aire*^{*-*/*-*} rats received from week 2 of age intraperitoneal injections of the mouse anti-rat CD45RC MAb (clone OX22, 1.5 mg/kg twice per week) up to week 20 when the animals were sacrificed.

We observed that 100% of Aire-deficient rats spontaneously developed alopecia and skin depigmentation in isotype control treated animals (Fig 7A top row), symptoms that correlate with a severe auto-immune disease and that are clinical manifestations regularly found in APECED patients. In contrast, Aire-deficient rats treated with anti-CD45RC mAb did not develop alopecia and skin depigmentation (Fig 7A bottom row), had a bigger thymus size (Fig. 7B) and a normal body weight (Fig. 7C), indicating a normal growth, a preserve thymus structure and altogether a decreased auto-immune disease.

Administration of a mouse anti rat-CD45RC monoclonal antibody from week 2 of age resulted in strong depletion of T CD8⁺ and CD4⁺ CD45RC^{high} cells in both spleen and lymph node in *Aire-l-* rats compared to rats treated with isotype control and untreated wt rats (Fig. 8).

Numbers of other major leukocyte populations (macrophages, B cells and NK cells) were unchanged (data not shown).

### REFERENCES

**PATENTS**

| | | |
|---|---|---|
| WO00/42072 | US 5,877,397; | US 5,635,483 |
| WO2004/029207 | US 5,661,016; | US 5,780,588 |
| WO2004/063351 | US 5,814,318; | US 6,214,345 |
| WO2004/074455 | US 5,874,299; | WO02088172 |
| WO00/42072 | US 5,770,429; | WO2004010957 |
| WO02/060919 | US 5,545,807; | WO2005081711 |
| WO2010/045193 | WO 92/03918; | WO2005084390 |
| WO2010/106180 | WO 93/12227; | WO2006132670 |
| WO99/51642 | WO 94/25585; | WO03026577 |
| WO2004074455 | WO 97/13852; | WO200700860 |
| US 4,946,778 | WO 98/24884; | WO207011968 |
| WO2016016442 | WO 99/45962 | WO205082023 |
| US 5,500,362 | WO 01/14424 | WO 89/12624 |
| US 5,821,337 | WO 02/43478 | WO 2012059882 |
| WO2008119353 | US 5,939,598; | WO 2016/091891 |
| WO 2011131746 | US 6,075, 181; | |
| US 5,591,669, | US 6,114,598; | |
| US 5,598,369, | US 6,150,584; | |
| US 5,545,806, | US 6,162,963 | |
| US 5,545,807, | WO 02/092812 | |
| US 6,150,584 | WO 2008/151081 | |
| US 5,569,825; | WO 2014/140322 | |
| US 5,625,126; | EP1176195A1 | |
| US 5,633,425; | WO01/77181 | |
| US 5,789,650; | WO2012/041768 | |

### NON-PATENTS

Almagro et al. Frontiers in Bioscience 13, 1619-1633, January 1, 2008.
Alman et al. (2005). Journal of pediatric orthopedics 25, 554-556.
Bruggermann et al., Year in Immuno., 7:33 (1993);
Chen et al., Embo J., vol. 12, 1993, pages 821 - 830
Chen et al., International Immunology, vol. 5, 1993, pages 647 - 656
Choi et al. (1993) Nature Genetics 4: 1 17-123
Clemens et al. Hum. Gene Ther. 1995;6:1477-1485.
Dall'Acqua et al. 2002, J Immunol.;169:5171-80.
Dall'Acqua et al. 2006, J. Biol. Chem.;281:23514-24. (a)
Dall'Acqua et al. J Immunol 2006; 177:1129-1138. (b)
Duchosal et al. Nature 355:258 (1992);
Fishwild et al., Nature Biotechnology, vol. 14, 1996, pages 845 - 851
Harding and Lonberg, Ann. N Y. ACAD. SCI., vol. 764, 1995, pages 536 - 546
Harper et al. Nat. Med. 2002;8:253-261.
Hinton et al. 2004, J Biol Chem.;279:6213-6.
Hoogenboom et al., J. Mol. Biol., 227:381 (1991);
Idusogie EE et al. J Immunol. 2001; 166:2571-5;
Jakobovits et al., Proc. Natl. Acad. Sci. USA. 90:2551 (1993) (a);
Jakobovits et al., Nature, 362:255-258 (1993) (b);
Jones et al. Nature, 321: 522-525, 1986;
Kuroiwa et al., Nature Biotechnology, vol. 20, 2002, pages 889 - 894
Labrijn et al. (2017); Scientific Reports 7, Article number: 2476
Lai Y. et al. J. Clin. Invest. 2009;119:624-635.
Larcher et al. (2014). PLoS ONE 9, e110371.
Lazar, G. A., et al. Proc Natl Acad Sci USA. 103(11): 4005-10 (2006).
Lonberg et al., nature, vol. 368, no. 6474, 1994a, pages 856 - 859;
Lonberg: "Handbook of Experimental Pharmacology", vol. 113, 1994b, pages: 49 - 101;
Lonberg, N. and Huszar, D. (1995) Intern. Rev. Immunol. 13: 65-93
Marks et al., J. Mol. Biol., 222:581- 5 597 (1991);
Moore GL. et al. mAbs 2:2, 181-189; March/April, 2010;
Odom et al. , Mol Ther. 2011;19:36-45.
Riechmann et al. Nature, 332: 323-327, 1988,
Rosenberget al. (2015). Sci Transl Med 7, 299rv294.
Shields RL, et al. J Biol Chem. 2001 Mar 2;276(9):6591-604.
Spickett et al., J EXP MED, vol. 158, 1983, pages 795 - 810,
Taylor et al., International immunology, vol. 6, 1994, pages 579 - 591
Taylor et al., Nucleic acids research, vol. 20, 1992, pages 6287 - 6295
Tomizuka et al., Proc. Natl. Acad. Set usa, vol. 97, 2000, pages 722 - 727
Tuaillon et al., J. Immunol., vol. 152, 1994, pages 2912 - 2920
Tuaillon et al., Proc. Natl. ACAD. SCI. USA, vol. 90, 1993, pages 3
Villalta et al.; Sci Transl Med. 15 octobre 2014 (6(258):258ra142);
Vaughan et al. Nature Biotech 14:309 (1996);
Verhoeyen et al. Science, 239: 1534-1536, 1988;

## Claims

1. Anti-CD45RC antibody for use in the treatment of monogenic diseases, wherein genes involved in said monogenic diseases are:
- genes which are not associated with immune function but whose deficiency is associated with inflammation and/or immune reactions, such as genes deficient in the following diseases: Duchenne muscular dystrophy (DMD), cystic fibrosis, lysosomal diseases and alpha1-antitrypsin deficiency; or
- genes involved in the immune system and whose deficiency generates inflammation and/or autoimmune reactions, such as genes deficient in the following diseases: T-cell primary immunodeficiencies such as IPEX (immunodysregulation polyendocrinopathy enteropathy X-linked syndrome), APECED (autoimmune polyendocrinopathy-candidiasis-ectodermal dystrophy), B cell primary immunodeficiencies, Muckle-Wells syndrome, mixed autoinflammatory and autoimmune syndrome, NLRP12-associated hereditary periodic fever syndrome, and tumor necrosis factor receptor 1 associated periodic syndrome.

2. Anti-CD45RC antibody for use according to claim 1, wherein said anti-CD45RC antibody is a monoclonal antibody.

3. Anti-CD45RC antibody for use according to claim 1 or 2, wherein said anti-CD45RC antibody is an anti-human CD45RC monoclonal antibody.

4. Anti-CD45RC antibody for use according to any one of claims 1 to 3, wherein said anti-CD45RC antibody is a chimeric antibody or a bispecific antibody or a humanized antibody or a fully human antibody.

5. Anti-CD45RC antibody for use according to any one of claims 1 to 4, wherein said anti-CD45RC antibody depletes T CD45RC^{high} cells.

6. Anti-CD45RC antibody for use according to claim 5, wherein said anti-CD45RC antibody is a multispecific antibody comprising a first antigen binding site directed against CDR45RC and at least one second antigen binding site directed against an effector cell able to mediate depletion of T CD45RC^{high} cells through direct binding, antibody-dependent cell-mediated cytotoxicity (ADCC), complement dependent cytotoxicity (CDC), and/or antibody-dependent phagocytosis.

7. Anti-CD45RC antibody for use according to anyone of claims 1 or 6, wherein said anti-CD45RC antibody is conjugated to a cytotoxic moiety.

8. Anti-CD45RC antibody for use according to any one of claims 1 to 7, wherein said anti-CD45RC antibody is used in combination with a pharmaceutically acceptable carrier or excipient or vehicle.

9. Anti-CD45RC antibody for use according to any one of claims 1 to 8, wherein said anti-CD45RC antibody is used in combination with an immunosuppressive and/or anti-inflammatory drug selected from corticoids such as glucocorticoids selected from dexamethasone, betamethasone, betamethasone-17-valerate, triamcinolone, triamcinolone acetonide, fluocinolone acetonide, fluocinonide, cortisone, hydrocortisone, prednisone, methylprednisolone, desonide, budesonide and deflazacort, preferentially prednisolone or deflazacort.

10. Anti-CD45RC antibody for use according to any one of claims 1 to 9, wherein said anti-CD45RC antibody is used in combination with gene therapy or cell therapy.

11. Anti-CD45RC antibody for use according to claim 10, wherein said gene therapy or cell therapy is used before or after the use of said anti-CD45RC antibody, preferentially before the use of said anti-CD45RC antibody.

12. Anti-CD45RC antibody for use according to any one of claims 1 to 11, wherein said monogenic disease is selected from DMD, cystic fibrosis and lysosomal storage diseases, preferably DMD.

13. Anti-CD45RC antibody for use according to any one of claims 1 to 11, wherein said monogenic disease is selected from T-cell primary immunodeficiencies such as IPEX (immunodysregulation polyendocrinopathy enteropathy X-linked syndrome), APECED (autoimmune polyendocrinopathy-candidiasis-ectodermal dystrophy), B cell primary immunodeficiencies, Muckle-Wells syndrome, mixed autoinflammatory and autoimmune syndrome, NLRP12-associated hereditary periodic fever syndrome, and tumor necrosis factor receptor 1 associated periodic syndrome, and is preferably APECED.

14. A pharmaceutical composition comprising an anti-CD45RC antibody as defined in any one of the claims 1 to 8 and an immunosuppressive and/or anti-inflammatory drug selected from corticoids such as glucocorticoids selected from dexamethasone, betamethasone, betamethasone-17-valerate, triamcinolone, triamcinolone acetonide, fluocinolone acetonide, fluocinonide, cortisone, hydrocortisone, prednisone, methylprednisolone, desonide, budesonide and deflazacort, preferentially prednisolone or deflazacort.

15. Product containing an anti-CD45RC antibody or a composition comprising an anti-CD45RC antibody as defined in anyone of the claims 1 to 8, and an immunosuppressive and/or an anti-inflammatory drug as defined in claim 14, and/or an adeno-associated virus or lentivirus containing at least one mini-dystrophin gene, as a combined preparation for simultaneous, separate or sequential use in the treatment of a monogenic disease.
